(19) Europäisches Patentamt
European Patent Office
Office européen des brevets

(11) Veröffentlichungsnummer: **0 279 201**
**A2**

# (12) EUROPÄISCHE PATENTANMELDUNG

(21) Anmeldenummer: **88100823.9**

(51) Int. Cl.4: **C07C 131/00**

(22) Anmeldetag: **21.01.88**

(30) Priorität: **27.01.87 DE 3702283**

(43) Veröffentlichungstag der Anmeldung:
**24.08.88 Patentblatt 88/34**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gayer, Herbert, Dr.**
**Alfred-Delp-Strasse 4**
**D-4019 Monheim(DE)**
Erfinder: **Jelich, Klaus, Dr.**
**Pahlkestrasse 5**
**D-5600 Wuppertal 1(DE)**

## (54) Verfahren zur Herstellung von 2-Cyano-2-oximono-acetamid-Derivaten.

(57) Ein neues Verfahren zur Herstellung von bekannten 2-Cyano-2-oximino-acetamid-Derivaten der Formel (I)

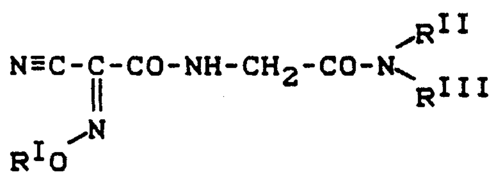

(I)

indem man 2-Cyano-2-oximino-acetamide der Formel (II)

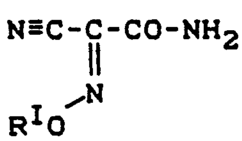

(II)

mit einer Base B, gegebenenfalls in Gegenwart eines Verdünnungsmittels, bei Temperaturen zwischen 0°C und 100°C umsetzt und die entstehenden Salze der Formel (III)

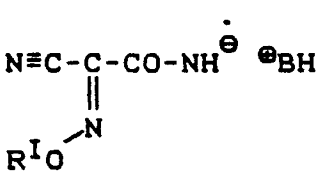

(III)

direkt oder gegebenenfalls nach Zwischenisolierung mit einem Alkylierungsmittel der Formel (IV)

EP 0 279 201 A2

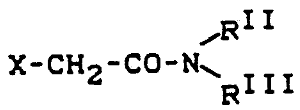

(IV)

gegebenenfalls in Gegenwart eines Verdünnungsmittels bei Temperaturen zwischen 0°C und 150°C umsetzt, wobei in den Formel (I) bis (IV) die jeweiligen Reste $R^I$ bis $R^{III}$, B und X die in der Beschreibung gegebenen Bedeutungen haben.

## Verfahren zur Herstellung von 2-Cyano-2-oximino-acetamid-Derivaten

Die vorliegende Erfindung betrifft ein neues Verfahren zur Herstellung von bekannten 2-Cyano-2-oximino-acetamid-Derivaten, welche fungizide Eigenschaften besitzen.

Es ist bereits bekannt, daß man 2-Cyano-2-oximino-acetamid-Derivate herstellen kann, indem man beispielsweise

a) Säurechloride mit Aminen in Gegenwart eines inerten organischen Lösungsmittels, wie beispielsweise Ether oder Toluol, und in Gegenwart eines organischen oder anorganischen Säurebindemittels, wie beispielsweise Triethylamin oder Natriumcarbonat, bei Temperaturen zwischen 0 und 120°C gemäß dem folgenden Reaktionsschema umsetzt:

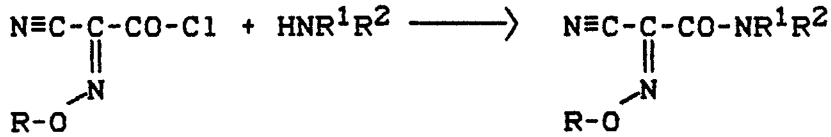

wobei die Rests z. B. bedeuten:
R = Alkyl, Alkenyl, Alkinyl oder gegebenenfalls subst. Phenylalkyl,
$R^1$ = H, Alkyl, und
$R^2$ = gegebenenfalls subst. Alkyl mit breiter Variation der Substituenten,
oder

b) Ester mit Aminen in Gegenwart eines inerten organischen Lösungsmittels, wie beispielsweise Alkohole oder Dimethylformamid, bei Temperaturen zwischen 0 und 150°C gemäß dem folgenden Reaktionsschema umsetzt:

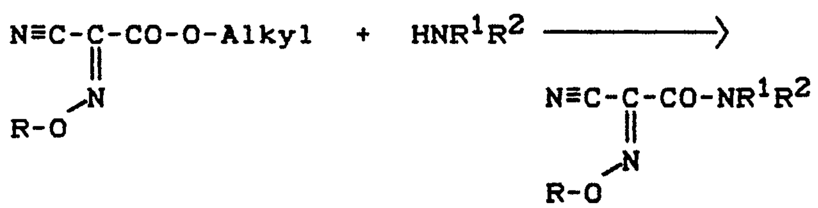

wobei die Reste z. B. bedeuten:
R = Alkyl, Alkenyl, Alkinyl oder gegebenenfalls subst. Phenylalkyl,
$R^1$ = H, Alkyl und
$R^2$ = gegebenenfalls subst. Alkyl mit breiter Variation der Substituenten,
oder

c) Oxime zunächst mit einer Base, wie beispielsweise Natriumethylat, in Gegenwart eines inerten organischen Lösungsmittels, wie beispielsweise Ethanol oder Acetonitril, bei Temperaturen zwischen 0 und 150°C umsetzt und das entstehende Salz in üblicher Weise alkyliert gemäß dem folgenden Reaktionsschema:

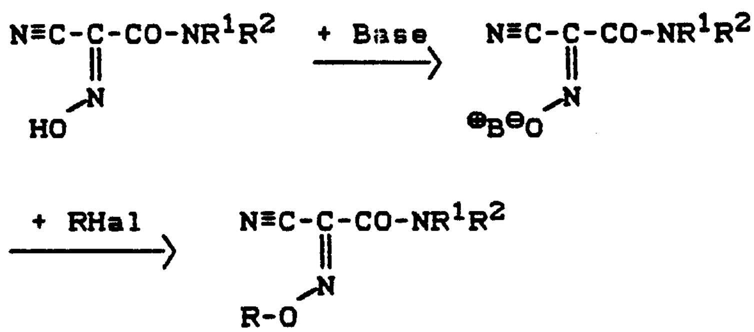


wobei die Reste z. B. bedeuten:

R = Alkyl, Alkenyl, Alkinyl, gegebenenfalls subst. Phenylalkyl,
$R^1$ = H, Alkyl,
$R^2$ = gegebenenfalls subst. Alkyl mit breiter Variation der Substituenten und
Hal = Halogen
oder

d) Acetamid-Derivate zunächst mit einer starken Base, wie beispielsweise Natriumhydrid, in Gegenwart eines inerten organischen Lösungsmittels, wie beispielsweise Dimethylformamid, bei Temperaturen zwischen -30 und +100°C umsetzt; und anschließend, gegebenenfalls unter Isolierung des entstehenden Salzes, in üblicher Weise alkyliert gemäß dem folgenden Reaktionschema:

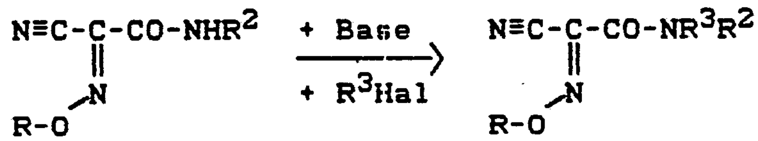


wobei die Reste z. B. bedeuten:
R = Alkyl, Alkenyl, Alkinyl, gegebenenfalls subst. Phenylalkyl,
$R^3$ = Cyanalkyl, Alkenyl, Alkinyl,
$R^2$ = gbf. subst. Alkyl mit breiter Variation der Substituenten und
Hal = Halogen (vgl. hierzu z.B. die Angaben gemäß DE-OS 23 12 956, EP O 201 999 und DE-OS 35 21 131).

Diese Verfahrensvarianten zeigen jedoch eine Reihe von Nachteilen. So sind die für das Verfahren (a) benötigten Säurechloride problematisch hinsichtlich ihrer Handhabung, da die Gefahr der Explosivität besteht. Die für das Verfahren (b) benötigten Ester lassen sich, vor allem in größerem Maßstab, nur sehr - schwer in reiner Form erhalten. Proleme bei der Reinigung treten auch bei dem Verfahren (c) auf, hier bei der Isolierung der Endprodukte. Das Verfahren (d) liefert nur disubstituierte Amide entsprechend der Beschreibung. Dabei ist die Handhabung der zur Protonierung der Acetamid-Derivate mit $R^2$ = Alkyl bevorzugt angewandten Basen Natriumhydrid und Lithiumdiisopropylamid nicht ungefährlich.

Es wurde gefunden, daß man die bekannten 2-Cyano-2-oximino-acetamid-Derivate der Formel (I)

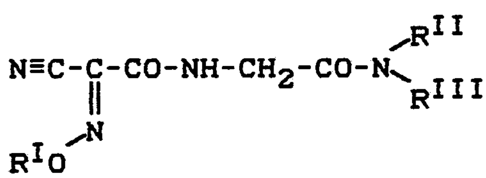
(I)

in welcher
$R^I$ für Alkyl, Alkenyl, Alkinyl, Cyanalkyl, Azolylalkyl, gegebenenfalls substituiertes Phenylalkyl oder für gegebenenfalls substituiertes Cycloalkyl steht;
$R^{II}$ für Wasserstoff oder Alkyl steht;
$R^{III}$ für Wasserstoff, Alkyl, Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl, gegebenenfalls substituiertes Cycloalkyl, für einen Acylamino-Rest oder für die Gruppierung
$-N=CH-R^{IV}$ steht, wobei
$R^{IV}$ für Alkyl oder gegebenenfalls substituiertes Phenyl steht,
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann,
erhält, wenn man 2-Cyano-2-oximino-acetamide der Formel (II)

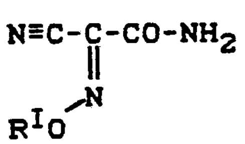
(II)

in welcher
$R^I$ die oben angegebene Bedeutung hat,
mit einer Base B, gegebenenfalls in Gegenwart eines Verdünnungsmittels, bei Temperaturen zwischen 0°C und 100°C umsetzt und die entstehenden Salze der Formel (III)

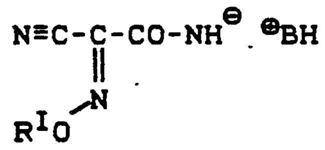

(III)

in welcher
$R^I$ die oben angegebene Bedeutung hat und
B für ein Äquivalent einer organischen oder anorganischen Base steht,
direkt oder gegebenenfalls nach Zwischenisolierung mit einem Alkylierungsmittel der Formel (IV)

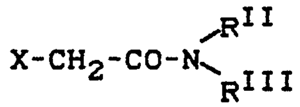

(IV)

in welcher
$R^{II}$ und $R^{III}$ die oben angegebene Bedeutung haben und
X für Halogen, Methan-oder p-Toluolsulfonat oder Methylsulfat steht,
gegebenenfalls in Gegenwart eines Verdünnungsmittels bei Temperaturen zwischen 0°C und 150°C umsetzt.

Es ist als überraschend zu bezeichnen, daß das erfindungsgemäße Verfahren so breit anwendbar ist. Zu erwarten war durchaus eine Selbstkondensation des Molekülteils

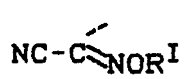

der 2-Cyano-2-oximino-acetamide der Formel (II) mit dem Carbamoyl-Teil $-CO-NH_2$ der Acetamide der Formel (II) bzw. mit dem entsprechenden Teil $-CO-NH^{\ominus}\ BH^{\oplus}$ der Salze der Formel (III), Außerdem mußte auch mit einer Doppelalkylierung der Acetamide der Formel (II) gerechnet werden.

Das erfindungsgemäße Verfahren hat den Vorteil, daß es in den Ausgangsstoffen der Formel (II) leicht zugänglich und einfach in reiner Form darstellbare Verbindungen besitzt (vgl. z.B. Chem. Ber. 54, 1342 (1921)). Die Endprodukte der Formel (I) sowie die Vorprodukte der Formel (II) können in den geometrischen Isomeren E und Z bzw. als Isomerengemische vorkommen.

Die nach dem erfindungsgemäßen Verfahren erhältlichen 2-Cyano-2-oximino-acetamid-Derivate sind durch die Formel (I) allgemein definiert. Bevorzugt sind Verbindungen der Formel (I), in denen
$R^I$ für geradkettiges oder verzweigtes Alkyl mit 1 bis 8 Kohlenstoffatomen, Alkenyl oder Alkinyl mit jeweils 2 bis 4 Kohlenstoffatomen im Alkylteil, für Cyanalkyl mit 1 bis 4 Kohlenstoffatomen, für 1,2,4-Triazol-1-ylalkyl und Pyrazol-1-ylalkyl mit jeweils 1 bis 6 Kohlenstoffatomen im Alkylteil, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seien: Halogen, Cyano, Nitro, Hydroxy, Alkyl, Alkoxy, Alkylthio, Alkylsulfinyl und Alkylsulfonyl mit jeweils 1 bis 4 Kohlenstoffatomen und gegebenenfalls einfach bis dreifach, gleich oder verschieden durch Halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien;
$R^{II}$ für Wasserstoff oder geradkettiges oder verzweigtes Alkyl mit 1 bis 4 Kohlenstoffatomen steht;
$R^{III}$ für Wasserstoff, geradkettiges oder verzweigtes Alkyl mit 1 bis 4 Kohlenstoffatomen, für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4

Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^I$ bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil beziehungsweise im Alkoxyteil, außerdem für Aminocarbonylamino, Alkylaminocarbonylamino oder Dialkylaminocarbonylamino mit jeweils 1 bis 4 Kohlen stoffatomen in den einzelnen Alkylteilen oder für Formylamino oder für die Gruppierung

-N = CH-$R^{IV}$ steht, wobei

$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei $R^I$ bereits genannten Phenylsubstituenten in Frage kommen,

oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl, Alkylaminocarbonyl oder Dialkylaminocarbonyl mit jeweils 1 bis 4 Kohlenstoffatomen in den einzelnen Alkylteilen substituiert sein kann; und

X für Chlor, Brom, lod, Methansulfonat, p-Toluolsulfonat oder Methylsulfat steht.

Besonders bevorzugt sind diejenigen Verbindungen der Formel (I), in denen

$R^I$ für geradkettiges oder verzweigtes Alkyl mit 1 bis 4 Kohlenstoffatomen, Allyl, Propargyl, Cyanmethyl, Cyanethyl, für 1,2,4-Triazol-1-ylalkyl und Pyrazol-1-ylalkyl mit jeweils 1 bis 4 Kohlenstoffatomen im Alkylteil, für gegebenenfalls im Phenylteil einfach oder zweifach, gleich oder verschieden substituiertes Phenylalkyl mit 1 oder 2 Kohlenstoffatomen im Alkylteil steht, wobei als Substituenten insbesondere genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht;

$R^{II}$ für Wasserstoff, Methyl oder Ethyl steht;

$R^{III}$ für Wasserstoff, Methyl, Ethyl, n-Propyl, i-Propyl, n-Butyl, i-Butyl, sec.-Butyl, tert.-Butyl, Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanalkyl mit 1 bis 4 Kohlenstoffatomen im Alkylteil; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^I$ bereits genannten Phenylsubstituenten in Frage kommen; für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl, für Alkylcarbonylamino oder Alkoxycarbonylamino mit jeweils 1 oder 2 Kohlenstoffatomen im Alkylteil beziehungsweise im Alkoxyteil, für Aminocarbonylamino, Methylaminocarbonylamino, Ethylaminocarbonylamino, Dimethylaminocarbonylamino, Diethylaminocarbonylamino, Methylethylaminocarbonylamino sowie für Formylamino oder für die Gruppierung

-N = CH-$R^{IV}$ steht, wobei

$R^{IV}$ für Methyl, Ethyl, für gegebenenfalls ein-oder zweifach, gleich oder verschieden substituiertes Phenyl steht, wobei als Substituenten die bei $R^I$ bereits vorzugsweise genannten Phenylsubstituenten in Frage kommen; oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteroatoms enthalten kann, wie insbesondere Piperidin, Pyrollidin, Morpholin oder Piperazin und gegebenenfalls durch Cyano, Fluor, Chlor, Brom, lod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann; und

X für Chlor oder Brom steht.

Verwendet man beispielsweise 2-Cyano-2-methoximino-acetamid als Ausgangsstoff, Kalium-tert.-butylat als Base und Chloressigsäurediethylamid als Alkylierungsmittel so kann der Verlauf des erfindungsgemäßen

Verfahrens durch das folgende Formelschema veranschaulicht werden:

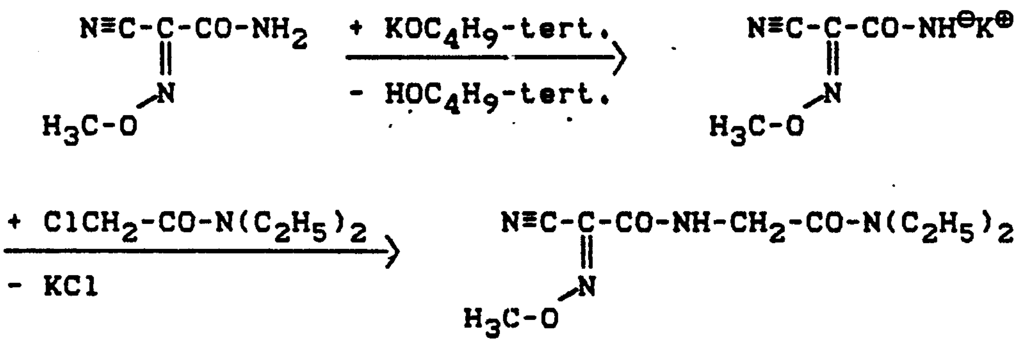


Die bei dem erfindungsgemäßen Verfahren als Ausgangsstoffe zu verwendenden 2-Cyano-2-oximino-acetamide sind durch die Formel (II) allgemein definiert. Bevorzugt sind Verbindungen der Formel (II), in denen

$R^1$ für geradkettiges oder verzweigtes Alkyl mit bis 8 Kohlenstoffatomen, Alkenyl oder Alkinyl mit jeweils 2 bis 4 Kohlenstoffatomen im Alkylteil, für Cyanalkyl mit 1 bis 4 Kohlenstoffatomen, für 1,2,4-Triazol-1-ylalkyl und Pyrazol-1-ylalkyl mit jeweils 1 bis 6 Kohlenstoffatomen im Alkylteil, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seien: Halogen, Cyano, Nitro, Hydroxy, Alkyl, Alkoxy, Alkylthio, Alkylsulfinyl und Alkylsulfonyl mit jeweils 1 bis 4 Kohlenstoffatomen und gegebenenfalls einfach bis dreifach, gleich oder verschieden durch Halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien.

Besonders bevorzugte Ausgangsstoffe sind diejenigen Verbindungen der Formel (II), in denen

$R^1$ für geradkettiges oder verzweigtes Alkyl mit 1 bis 4 Kohlenstoffatomen, Allyl, Propargyl, Cyanmethyl, Cyanethyl, für 1,2,4-Triazol-1-ylalkyl und Pyrazol-1-ylalkyl mit jeweils 1 bis 4 Kohlenstoffatomen im Alkylteil, für gegebenenfalls im Phenylteil einfach oder zweifach, gleich oder verschieden substituiertes Phenylalkyl mit 1 bis 2 Kohlenstoffatomen im Alkyl teil steht, wobei als Substituenten insbesondere genannt seien: Fluor, Chlor, Cyano, Nitro, Hydroxy, methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht.

Die 2-Cyano-2-oximino-acetamide der Formel (II) sind bekannt (vergleiche z.B. Chem. Ber. 54, 1342 (1921)) bzw. können sie in allgemein bekannter Art und Weise erhalten werden, indem man beispielsweise Alkalimetallsalze von 2-Cyano-2-oximino-acetamiden der Formel (IIa)

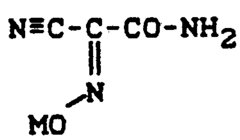
(IIa)

in welcher

M für ein Alkalimetall, insbesondere Natrium, steht,

mit einem Alkylierungsmittel, wie z.B. Dimethylsulfat, in Gegenwart eines inerten, organischen Lösungsmittels, wie z.B. Aceton, bei Temperaturen zwischen 0°C und 60°C umsetzt.

Die Alkalimetallsalze (vgl.auch Chem. Ber. 42, 738 (1909)) erhält man besonders vorteilhaft, indem man Cyanacetamid mit einem Alkylnitrit, wie beispielsweise Isoamylnitrit, und einem Alkalialkoholat, wie beispielsweise Natriumethylat, in Gegenwart eines Alkohols, wie beispielsweise Ethanol, bei Temperaturen zwischen 0°C und 20°C umsetzt.

Die außerdem für das erfindungsgemäße Verfahren als Ausgangsstoffe zu verwendenden Basen sind durch den Buchstaben B allgemein definiert. B steht vorzugsweise für übliche anaorganische und organische Basen, wie insbesondere Alkalimetallalkoholate, wie beispielsweise Kalium-tert.-butylat; Alkalimetallhydroxide und -carbonate, wie beispielsweise Natriumhydroxid, Kaliumhydroxid und Kaliumcarbonat: sowie für Tetraalkylammonium-bzw. Benzyltrialkylammonium-hydroxide und -alkoholate, wie beispielsweise Tetramethylammoniumhydroxid und -alkoholat, Tetrabutylammoniumhydroxid und -alkoholat oder Benzyltriethylammoniumhydroxid und -alkoholat.

Die Basen B sind allgemein bekannte Verbindungen der organischen Chemie.

Die weiterhin für das erfindungsgemäße Verfahren als Ausgangsstoffe zu verwendenden Alkylierungsmittel sind durch die Formel (IV) allgemein definiert. Bevorzugt sind Verbindungen der Formel (IV), in denen $R^{II}$ für Wasserstoff oder geradkettiges oder verzweigtes Alkyl mit 1 bis 4 Kohlenstoffatomen steht;

$R^{III}$ für Wasserstoff, geradkettiges oder verzweigtes Alkyl mit 1 bis 4 Kohlenstoffatomen, für Alkenyl oder Alkinyl it jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit 1 bis 4 Kohlenstoffatomen in den einzelnen Alkylteilen, für Alkoxycarbonylalkyl mit jeweils 1 bis 4 Kohlenstoffatomen im Alkoxyteil und im Alkylteil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^{I}$ bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil beziehungsweise im Alkoxyteil, außerdem für Aminocarbonylamino, Alkylaminocarbonylamino und Dialkylaminocarbonylamino mit jeweils 1 bis 4 Kohlenstoffatomen in den einzelnen Alkylteilen sowie für Formylamino oder für die Gruppierung

$-N=CH-R^{IV}$ steht, wobei

$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei $R^{I}$ bereits genannten Phenylsubstituenten in Frage kommen,

oder

$R^{II}$ und $R^{III}$ gemeinsam dem Stickstoffatom, an das sie gebunden sind, für einen 5-oder 6-gliedrigen Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl sowie Alkylaminocarbonyl und Dialkylaminocarbonyl mit jeweils 1 bis 4 Kohlenstoffatomen in den einzelnen Alkylteilen substituiert sein kann; und

X für Chlor, Brom, Iod, Methansulfonat, p-Toluolsulfonat oder Methylsulfat steht.

Besonders bevorzugte Ausgangsstoffe sind diejenigen Verbindungen der Formel (IV), in denen

$R^{II}$ für Wasserstoff, Methyl oder Ethyl steht;

$R^{III}$ für Wasserstoff, Methyl, Ethyl, n-Propyl, i-Propyl, n-Butyl, i-Butyl, sec.-Butyl, tert.-Butyl, Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanalkyl mit 1 bis 4 Kohlenstoffatomen im Alkylteil; ferner für gegebenenfalls im Phenylteil einfach oder zweifach, gleich oder verschieden substituiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^{I}$ hereits genannten Phenylsubstituenten in Frage kommen; für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl, für Alkylcarbonylamino oder Alkoxycarbonylamino mit jeweils 1 oder 2 Kohlenstoffatomen im Alkylteil beziehungsweise im Alkoxyteil, für Aminocarbonylamino, Methylaminocarbonylamino, Ethylaminocarbonylamino, Dimethylaminocarbonylamino, Diethylaminocarbonylamino, Methylethylaminocarbonylamino sowie für Formylamino oder für die Gruppierung

$-N=CH-R^{IV}$ steht, wobei

$R^{IV}$ für Methyl, Ethyl, für gegebenenfalls ein-oder zweifach, gleich oder verschieden substituiertes Phenyl steht, wobei als Substituenten die bei $R^{I}$ bereits vorzugsweise genannten Phenylsubstituenten in Frage kommen; oder

$R^{II}$ und $R^{III}$ gemeinsam mit dem Stickstoffatom, an das sie gebunden sind, für einen 5-oder 6-gliedrigen Heterocyclus stehen, der gegebenenfalls Sauerstoff oder Stickstoff als weitere Heteroatome enthalten kann, wie insbesondere Piperidin, Pyrollidin, Morpholin oder Piperazin und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann; und

X für Chlor oder Brom steht.

Die Alkylierungsmittel der Formel (IV) sind bekannt bzw. lassen sie sich in bekannter Art und Weise erhalten.

Als Verdünnungsmittel kommen bei der Durchführung des erfindungsgemäßen Verfahrens alle unter den Reaktionsbedingungen inerten organischen Solventien in Betracht. Vor zugsweise verwendbar sind Ether, wie Diethylether, Diisopropylether, Tetrahydrofuran oder Dioxan; Ketone, wie Aceton oder Methylethylketon; ferner Acetonitril und Dimethylformamid.

Die Reaktionstemperaturen können bei der Durchführung des erfindungsgemäßen Verfahrens in einem größeren Bereich variiert werden. Bei der Salzbildung arbeitet man allgemein zwischen 0°C und 100°C, vorzugsweise zwischen 0°C und 30°C. Bei der anschließenden Alkylierung arbeitet man allgemein zwischen 0°C und 150°C, vorzugsweise zwischen 60°C und 120°C.

Bei der Durchführung des erfindungsgemäßen Verfahrens setzt man vorzugsweise äquimolare Mengen ein, gegebenenfalls einen Überschuß bis zu 1 Mol an Alkylierungsmittel der Formel (IV). Die Salze der Formel (III) können gegebenenfalls isoliert werden, es kann aber auch ohne Isolierung gearbeitet werden. In manchen Fällen erweist es sich als vorteilhaft, die Acetamide der Formel (II) zunächst zu einem Alkalimetallsalz der Formel (III) umzusetzen und dieses dann mit Hilfe von Tetraalkylammonium-bzw. Benzyltrialkylammoniumchloriden oder -bromiden in das entsprechende Ammoniumsalz zu überführen. Die Isolierung der Endprodukte der Formel (I) erfolgt in üblicher Weise.

Die nach dem erfindungsgemäßen Verfahren herstellbaren 2-Cyano-2-oximino-acetamid-Derivate der Formel (I) sind bekannt (vgl. DE-OS 35 21 131). Sie zeichnen sich durch sehr gute fungizide Eigenschaften aus.

Das erfindungsgemäße Verfahren wird durch die nachstehenden Beispiele veranschaulicht.

Beispiel 1

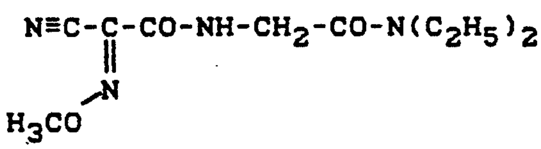

(I-1)

(Variante 1)

6,4 g (0,05 Mol) 2-Cyano-2-methoximino-acetamid (E-Isomeres) werden in 50 ml Tetrahydrofuran gelöst. Unter Kühlung tropft man zu dieser Lösung 5,7 g (0,05 Mol) Kalium-tert.-butylat, gelöst in 50 ml Tetrahydrofuran, so zu, daß die Temperatur 20°C nicht übersteigt. Man rührt 15 Minuten bei 10°C nach und filtriert das auskristallisierte Salz ab. Man erhält 7,6 g (92 % der Theorie = 0,046 Mol) Kalium-Salz des 2-Cyano-2-methoximino-acetamids, das in 50 ml Tetrahydrofuran suspendiert wird. Nach Zugabe von 6,9 g (0,046 Mol) Chloressigsäurediethylamid wird 1 Stunde unter Rückfluß erhitzt. Anschließend wird eingeengt und der Rückstand chromatographiert (Methylenchlorid/Aceton = 4/1).

Man erhält 4,6 g (38,3 % der Theorie) 2-Cyano-2-methoximino-essigsäure-diethylcarbamoylmethylamid (E-Isomeres) vom Schmelzpunkt 104-06°C.

(Variante 2)

2,3 g (0,01 Mol) Benzyltriethylammoniumchlorid in 10 ml Tetrahydrofuran werden mit 5 ml (0,01 Mol) einer 2-molaren Lösung von Natriummethylat in Methanol versetzt und im Vakuum bei einer Badtemperatur unter 40°C eingeengt. Der Rückstand wird mit einer Lösung von 1,3 g (0,01 Mol) 2-Cyano-2-methoximino-acetamid (E-Isomeres) in 20 ml Tetrahydrofuran bei Raumtemperatur versetzt. Man gibt 1,5 g (0,01 Mol) Chloressigsäurediethylamid zu und erhizt 30 Minuten unter Rückfluß. Nach dem Abziehen des Lösungsmittels und nach Versetzen mit 10 ml Eiswasser kristallisieren 1,22 g (50,7 % der Theorie) 2-Cyano-2-methoximino-essigsäure-diethylcarbamoylmethylamid (E-Isomeres) vom Schmelzpunkt 104-06°C aus.

<u>Herstellung der Ausgangsverbindung</u>

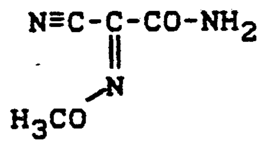

(II-1)

27 g (0,2 Mol) des Natriumsalzes von 2-Cyano-2-oximino-acetamid (E-Isomeres) und 25,3 g (0,2 Mol) Dimethylsulfat werden in 200 ml Aceton 10 Minuten unter Rückfluß erhitzt. Nach dem Abkühlen auf Raumtemperatur filtriert man 25 g des Natriumsalzes der O-Methylschwefelsäure ab. Das Aceton wird abgezogen und der Rückstand mit 100 ml Wasser 30 Minuten bei Raumtemperatur, dann 1 Stunde bei 0°C gerührt. Man filtriert ab und erhält 20,8 g (81,82 % der Theorie) 2-Cyano-2-methoximinoacetamid (E-Isomeres) vom Schmelzpunkt 168-169°C.

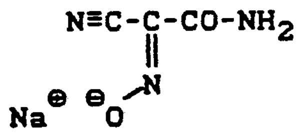

(IIa-1)

11,5 g (0,5 Mol) Natrium werden in 200 ml Ethanol gelöst. Diese Lösung tropft man bei einer Temperatur unter 20°C zu einer Mischung aus 42 g (0,5 Mol) Cyanacetamid, 64,4 g (0,55 Mol) Isoamylnitrit und 300 ml Ethanol. Man rührt eine Stunde bei Raumtemperatur und filtriert dann das Produkt ab.
Man erhält 60,1 g (89 % der Theorie) des Natriumsalzes von 2-Cyano-2-oximinoacetamid (E-Isomeres).

**Ansprüche**

1. Verfahren zur Herstellung von 2-Cyano-2-oximino-acetamid-Derivaten der Formel (I)

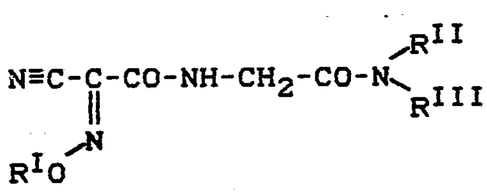

(I)

in welcher
$R^I$ für Alkyl, Alkenyl, Alkinyl, Cyanalkyl, Azolylalkyl, gegebenenfalls substituiertes Phenylalkyl oder für gegebenenfalls substituiertes Cycloalkyl steht;
$R^{II}$ für Wasserstoff oder Alkyl steht;
$R^{III}$ für Wasserstoff, Alkyl, Alkenyl, Alkinyl, Halogenalkyl, Alkoxyalkyl, Dialkylaminoalkyl, Alkoxycarbonylalkyl, Hydroxycarbonylalkyl, Aminocarbonylalkyl, Alkylaminocarbonylalkyl, Dialkylaminocarbonylalkyl, Cyanoalkyl, gegebenenfalls substituiertes Phenylalkyl, gegebenenfalls substituiertes Phenyl, gegebenenfalls substituiertes Cycloalkyl, für einen Acylamino-Rest oder für die Gruppierung
$-N=CH-R^{IV}$ steht, wobei
$R^{IV}$ für Alkyl oder gegebenenfalls substituiertes Phenyl steht,
oder
$R^{II}$ und $R^{III}$ gemeinsam mit dem Stickstoff-Atom, an das sie gebunden sind, für einen gegebenenfalls substituierten Heterocyclus stehen, der weitere Heteroatome enthalten kann,
dadurch gkennzeichnet, daß man 2-Cyano-2-oximino-acetamide der Formel (II)

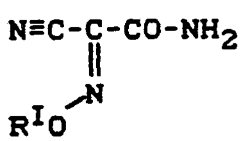

(II)

in welcher
$R^I$ die oben angegebene Bedeutung hat,
mit einer Base B, gegebenenfalls in Gegenwart eines Verdünnungsmittels, bei Temperaturen zwischen 0°C und 100°C umsetzt und die entstehenden Salze der Formel (III)

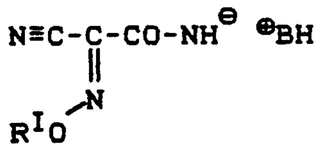

(III)

in welcher
$R^I$ die oben angegebene Bedeutung hat und
B für ein Äquivalent einer organischen oder anorganischen Base steht,
direkt oder gegebenenfalls nach Zwischenisolierung mit einem Alkylierungsmittel der Formel (IV)

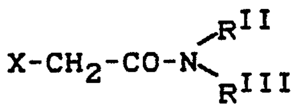

(IV)

in welcher
$R^{II}$ und $R^{III}$ die oben angegebene Bedeutung haben und
X für Halogen, Methan-oder p-Toluolsulfonat oder Methylsulfat steht,
gegebenenfalls in Gegenwart eines Verdünnungsmittels bei Temperaturen zwischen 0°C und 150°C umsetzt.

2. Verfahren gemäß Anspruch 1, worin in der Formel (I)
$R^I$ für geradkettiges oder verzweigtes Alkyl mit 1 bis 8 Kohlenstoffatomen, Alkenyl oder Alkinyl mit jeweils 2 bis 4 Kohlenstoffatomen im Alkylteil, für Cyanalkyl mit 1 bis 4 Kohlenstoffatomen, für 1,2,4-Triazol-1-ylalkyl oder Pyrazol-1-ylalkyl mit jeweils 1 bis 6 Kohlenstoff atomen im Alkylteil, für gegebenenfalls im Phenylteil einfach bis dreifach, gleich oder verschieden substituiertes Phenylalkyl mit 1 bis 4 Kohlenstoffatomen im Alkylteil steht, wobei als Substituenten vorzugsweise genannt seinen: Halogen, Cyano, Nitro, Hydroxy, Alkyl, Alkoxy, Alkylthio, Alkylsulfinyl und Alkylsulfonyl mit jeweils 1 bis 4 Kohlenstoffatomen und gegebenenfalls einfach bis dreifach, gleich oder verschieden durch Halogen substituiertes Phenyl; oder für gegebenenfalls einfach bis dreifach, gleich oder verschieden substituiertes Cycloalkyl mit 3 bis 7 Kohlenstoffatomen steht, wobei als Substituenten vorzugsweise Halogen und Alkyl mit 1 bis 4 Kohlenstoffatomen genannt seien.
$R^{II}$ für Wasserstoff oder geradkettiges oder verzweigtes Alkyl mit 1 bis 4 Kohlenstoffatomen steht;
$R^{III}$ für Wasserstoff, geradkettiges oder verzweigtes Alkyl mit 1 bis 4 Kohlenstoffatomen, für Alkenyl oder Alkinyl mit jeweils 2 bis 4 Kohlenstoffatomen, für Halogenalkyl mit 1 bis 4 Kohlenstoffatomen und 1 bis 5 gleichen oder verschiedenen Halogenatomen, für Alkoxyalkyl mit jeweils 1 bis 4 Kohlenstoffatomen im Alkoxyteil und im Alkylteil, für Dialkylaminoalkyl mit bis zu 4 Kohlenstoffatomen in den einzelnen Alkylteilen, für Alkoxycarbonylalkyl mit jeweils 1 bis 4 Kohlenstoffatomen im Alkoxyteil und im Alkyl teil, Hydroxycarbonylalkyl mit 1 bis 4 Kohlenstoffatomen im Alkylteil, Aminocarbonylalkyl, Alkylaminocarbonylalkyl oder Dialkylaminocarbonylalkyl mit jeweils 1 bis 4 Kohlenstoffatomen in jedem Alkylteil, für Cyanalkyl mit 1 bis 4 Kohlenstoffatomen im Alkylteil, gegebenenfalls im Phenylteil einfach bis dreifach, gleich oder verschieden substituiertes Phenylalkyl mit 1 bis 4 Kohlenstoffatomen im Alkylteil oder einfach bis dreifach, gleich oder verschieden substituiertes Phenyl steht, wobei als Substituenten jeweils die bei $R^I$ bereits genannten Phenylsubstituenten in Frage kommen, für gegebenenfalls gleich oder verschieden substituiertes Cycloalkyl mit 3 bis 7 Kohlenstoffatomen, wobei als Substituenten vorzugsweise Halogen und Alkyl mit 1 bis 4 Kohlenstoffatomen in Frage kommen, für Alkylcarbonylamino oder Alkoxycarbonylamino mit jeweils 1 bis 4 Kohlenstoffatomen im Alkylteil beziehungsweise im Alkoxyteil, außerdem für Aminocarbonylamino, Alkylaminocarbonylamino oder Dialkylaminocarbonylamino mit jeweils 1 bis 4 Kohlenstoffatomen in den einzelnen Alkylteilen, für Formylamino oder für die Gruppierung
$-N=CH-R^{IV}$ steht, wobei
$R^{IV}$ für geradkettiges oder verzweigtes Alkyl mit 1 bis 4 Kohlenstoffatomen oder gegebenenfalls einfach bis dreifach, gleich oder verschieden substituiertes Phenyl steht, wobei als Substituenten die bei $R^I$ bereits genannten Phenylsubstituenten in Frage kommen,
oder
$R^{II}$ und $R^{III}$ gemeinsama mit dem Stickstoffatom, an das sie gebunden sind, für einen 5-oder 6-gliedrigen

Heterocyclus stehen, der gegebenenfalls Sauerstoff oder Stickstoff als weitere Heteroatome enthalten kann und gegebenenfalls durch Cyano, Halogen, Alkyl mit 1 bis 4 Kohlenstoffatomen, Hydroxycarbonyl, Alkoxycarbonyl mit 1 bis 4 Kohlenstoffatomen im Alkoxyteil, Aminocarbonyl, Alkylaminocarbonyl oder Dialkylaminocarbonyl mit jeweils 1 bis 4 Kohlenstoffatomen in den einzelnen Alkylteilen substituiert sein kann; und X für Chlor, Brom, Iod, Methansulfonat, p-Toluolsulfonat oder Methylsulfat steht.

3. Verfahren gemäß Anspruch 1, worin in der Formel (I)
$R^I$ für geradkettiges oder verzweigtes Alkyl mit 1 bis 4 Kohlenstoffatomen, Allyl, Propargyl, Cyanmethyl, Cyanethyl, für 1,2,4-Triazol-1-ylalkyl oder Pyrazol-1-ylalkyl mit jeweils 1 bis 4 Kohlenstoffatomen im Alkylteil, für gegebenenfalls im Phenylteil einfach oder zweifach, gleich oder verschieden substituiertes Phenyl alkyl mit 1 oder 2 Kohlenstoffatomen im Alkylteil steht, wobei als Substituenten insbesondere genannt seien: Fluor, Chlor, Cyano, Nitro, Hydroxy, Methyl, Methoxy, Methylthio, Methylsulfinyl, Methylsulfonyl und gegebenenfalls einfach oder zweifach, gleich oder verschieden durch Fluor oder Chlor substituiertes Phenyl; ferner für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl steht,
$R^{II}$ für Wasserstoff, Methyl oder Ethyl steht;
$R^{III}$ für Wasserstoff, Methyl, Ethyl, n-Propyl, i-Propyl, n-Butyl, i-Butyl, sec.-Butyl, tert.-Butyl, Allyl, Propargyl, für Halogenalkyl mit 1 oder 2 Kohlenstoffatomen und 1 bis 3 gleichen oder verschiedenen Halogenatomen, wie Fluor-und Chloratomen, für Alkoxyalkyl mit jeweils 1 oder 2 Kohlenstoffatomen im Alkoxyteil und im Alkylteil, für Dialkylaminoalkyl mit 1 oder 2 Kohlenstoffatomen in den einzelnen Alkylteilen, für Alkoxycarbonylalkyl, Hydroxycarbonylalkyl, Aminocarbonylalkyl, Alkylaminocarbonylalkyl oder Dialkylaminocarbonylalkyl mit jeweils 1 oder 2 Kohlenstoffatomen in jedem Alkylteil, Cyanalkyl mit 1 bis 4 Kohlenstoffatomen im Alkylteil; ferner für gegebenenfalls im Phenylteil einfach oder zweifach, gleich oder verschieden substi tuiertes Phenylalkyl mit 1 oder 2 Kohlenstoffatomen im Alkylteil, für einfach oder zweifach, gleich oder verschieden substituiertes Phenyl steht, wobei als Substituenten jeweils die bei $R^I$ bereits genannten Phenylsubstituenten in Frage kommen; für jeweils gegebenenfalls einfach oder zweifach, gleich oder verschieden durch Fluor, Chlor oder Methyl substituiertes Cyclopropyl, Cyclobutyl, Cyclopentyl, Cyclohexyl oder Cycloheptyl, für Alkylcarbonylamino oder Alkoxycarbonylamino mit jeweils 1 oder 2 Kohlenstoffatomen im Alkylteil beziehungsweise im Alkoxyteil, für Aminocarbonylamino, Methylaminocarbonylamino, Ethylaminocarbonylamino, Dimethylaminocarbonylamino, Diethylaminocarbonylamino, Methylethylaminocarbonylamino, für Formylamino oder für die Gruppierung
$-N=CH-R^{IV}$ steht, wobei
$R^{IV}$ für Methyl, Ethyl, für gegebenenfalls ein-oder zweifach, gleich oder verschieden substituiertes Phenyl steht, wobei als Substituenten die bei $R^I$ bereits vorzugsweise genannten Phenylsubstituenten in Frage kommen; oder
$R^{II}$ und $R^{III}$ gemeinsam mit dem Stickstoffatom, an das sie gebunden sind, für Piperidin, Pyrollidin, Morpholin oder Piperazin stehen und gegebenenfalls durch Cyano, Fluor, Chlor, Brom, Iod, Methyl, Ethyl, i-Propyl, Hydroxycarbonyl, Methoxycarbonyl, Ethoxycarbonyl, Aminocarbonyl, Methylaminocarbonyl, Ethylaminocarbonyl, Dimethylaminocarbonyl, Diethylaminocarbonyl sowie Methylethylaminocarbonyl substituiert sein kann; und
X für Chlor oder Brom steht.

4. Verfahren gemäß Anspruch 1, worin die verwendete Base B ein Alkalimetallalkoholat, Alkalimetallhydroxid, Alkalimetallcarbonat, Tetraalkylammonium-oder Benzyltrialkylammoniumhydroxid oder -alkoholat ist.